# EUROPEAN PATENT SPECIFICATION

(11) **EP 2 036 601 B1**
(45) Date of publication and mention of the grant of the patent: **25.12.2019**
(21) Application number: 07730348.5
(22) Date of filing: 27.02.2007
(51) Int. Cl.: B01D 15/00, B01D 15/08, B01J 20/26, B01J 20/285, B01D 15/42, A23G 1/32, A23G 1/00, A23G 1/56, C07D 311/62

(54) **PROCESS FOR OBTAINING POLYPHENOL-RICH COCOA POWDER WITH A LOW FAT CONTENT**
VERFAHREN ZUM ERHALT VON POLYPHENOLREICHEM KAKAOPULVER MIT EINEM GERINGEN FETTGEHALT
PROCEDE D'OBTENTION DE CACAO EN POUDRE RICHE EN POLYPHENOLS ET A FAIBLE TENEUR EN MATIERE GRASSE

(30) Priority: 27.02.2006 ES 200600462
(43) Date of publication of application: 18.03.2009
(73) Proprietor: Naturex S.A., 84911 Avignon Cedex 09 (FR)
(72) Inventor: PASAMAR FERNÁNDEZ, María Angeles, E-46019 Valencia (ES); FRITZ KURT, Jaisli, CH-8589 Sitterdorf (CH); ARCOS PALACIOS, Francisco Javier, E-28700 San Sebastián del los Reyes - Madrid (ES); RAMÓN VIDAL, Daniel, E-46183 L'Eliana, Valencia (ES); CASTILLA ESCOBAR, Yolanda, E-46900 Valencia (ES); CIENFUEGOS-JOVEL-LANOS FERNANDEZ, Elena, E-46370 Chiva - Valencia (ES)
(74) Representative: Potter Clarkson
(86) International application number: PCT/ES2007/000107
(87) International publication number: WO 2007/096449

(56) References cited:
- WO-A1-2005/115160
- WO-A1-2005/115160
- CA-A1- 2 315 886
- DE-A1- 10 247 476
- US-A1- 2002 064 584
- US-A1- 2005 276 893
- US-B1- 6 194 020
- PORTER L J ET AL: "Cacao procyanidins: major flavanoids and identification of some minor metabolites", PHYTOCHEMISTRY, PERGAMON PRESS, GB, vol. 30, no. 5, 1 January 1991 (1991-01-01), pages 1657-1663, XP026631933, ISSN: 0031-9422, DOI: 10.1016/0031-9422(91)84228-K [retrieved on 1991-01-01]

## Description

### Field of the Art

The present invention relates to a process for obtaining cocoa powder with high polyphenol content and low fat content as defined in Claim 1. Also described herein is the cocoa extract obtained from said defatted material.

Due to the increasing interest and demand for new functional cocoa-derived polyphenol-rich food products, the development of industrial processes for elaborating said products is advantageous and suitable.

There are several factors affecting the polyphenol content of cocoa apart from the botanical varieties of cocoa. These factors include most of the steps involved in the industrial processes for manufacturing cocoa powder, including bean fermentation, drying, roasting, as well as the alkalization process.

The steps for fermenting, roasting and drying cocoa seeds are the most significant steps, since important chemical and structural changes occur, leading to a significant decrease in the total polyphenol content in cocoa seeds (Forsyth and Quesnel, 1963; Kim and Keeney, 1984; Hansen et. al. 1998).

The development of the characteristic cocoa color is due to the action of the polyphenol oxidase (PPO) enzyme, which is a metalloenzyme (0.2% Cu) with oxidoreductase activity. This enzyme acts by oxidizing the phenol compounds present in cocoa, giving rise to melanoidins (brown pigments) and consequently causing the degradation and reduction of polyphenolic substances.

Therefore, the PPO enzyme catalyzes two basic reactions throughout aerobic fermentation: the hydroxylation of monophenols to o-diphenols (monophenol oxidase enzyme), and the oxidation of the diphenol substrate to quinone (diphenol oxidase enzyme). In both reactions, PPO uses molecular oxygen as a co-substrate. The polymerization of o-benzoquinone then occurs, the melanoid polymers finally being developed.

### State of the Art

The most relevant state of the art is centered in processes in which cocoa seeds are processed to elaborate cocoa powder, preventing the reduction of the phenolic compounds initially present in fresh cocoa beans.

US patent 6485772 describes obtaining a cocoa powder with a polyphenol content of 7 to 9%, and with a less astringent taste than conventional cocoa. Cocoa beans from Ecuador and Venezuela are used as a starting product in said patent.

The process described in said patent comprises a series of sequential steps, consisting of shelling the beans, alkalizing the nib (shell-free cocoa beans), roasting the nib, grinding, pressing the paste to extract the fat and finally pulverizing the cocoa powder, so as to reach the objective which is to reduce the bitterness of cocoa without reducing the amount of polyphenols present in cocoa seeds.

US patent 6015913 describes the technique for elaborating a solid (cocoa-derived partially defatted powder) in which the polyphenol profile does not substantially change competed to that of the raw material.

The raw material in this process are fermented cocoa beans which are selected according to the variety of the bean and the fermentation level (evaluated according to the seed color), including both non-fermented seeds and fermented seeds.

The described process starts with a heat treatment of the cocoa beans by means of infrared rays at 100-110°C for the purpose of facilitating the separation of the shell from the nib.

The beans are then shelled, ground, partially defatted by an expeller and finally ground again. This cocoa powder can optionally be alkalized. The patent additionally describes methods for extracting polyphenols with solvents from the cocoa powder obtained.

US patent 6312753 is an extension of US 6015913. The description of the new patent includes the process for roasting cocoa beans. This patent details the procyanidin contents found in the end product.

US patent 20040096566 describes obtaining a polyphenol-rich cocoa extract, taking fresh cocoa beans obtained immediately after their extraction from the inside of cocoa pods as raw material.

Once the pulp and shell of the fresh seeds have been eliminated, the obtained nibs are ground in the presence of suitable solvents, allowing them to macerate in conditions facilitating the extraction of cocoa polyphenols. A filtration process with a subsequent distillation of the extract in order to recover the solvent is then described.

Patent WO 2005/115160 describes obtaining a polyphenol-rich cocoa extract taking unfermented cocoa beans as raw material, in which they are first subjected to a blanching treatment, after which drying, a particle size reduction, an extraction and a concentration are carried out.

US2002/064584 describes cocoa components that have enhanced levels of cocoa polyphenols, processes for producing the cocoa components while conserving a significant amount of the cocoa polyphenols, compositions containing the cocoa components or the cocoa polyphenols, and methods of using the cocoa components or the cocoa polyphenols for improving the health of a mammal are described.

Porter L et al, Phytochemistry, vol. 30, no.5, 1991 describes the major flavonoids and minor metabolites found in fresh, unfermented cocoa beans.

There is currently extensive patent and non-patent literature supporting the possible benefits of including cocoa in the diet due to the polyphenols it contains, specifically flavonoids and their specific class flavonols, which provide a beneficial effect in circulation (L.I. Meneen et al., "Consumption of foods rich in flavonoids is related to a decreased cardiovascular risk in apparently healthy French women", (2004) J.Nutr 13, 923-926), for the heart (M.G. Hertog et al., "Dietary antioxidant flavonoids and risk of coronary disease: the Zutphen Elderly Study", Lancet (1993) 342, 1007-1011), against cancer (M.G. Hertog et al., "Flavonoid intake and long-term risk of coronary heart disease and cancer in the seven countries study", Arch. Inter. Med. (1995) 155, 381-386), and they contribute to preventing neurodegenerative diseases anddiabetes mellitus(A. Scalbert et al., "Dietary polyphenols and the prevention of diseases", Crit. Rev. Food Sci. Nutr.(2005) 45, 287-306).

### Description of the Invention

The object of the invention relates to an alternative process to the state of the art for processing cocoa seeds, in which on one hand the reduction of the phenolic compounds initially present in fresh cocoa beans is prevented and on the other hand the fat content is reduced until achieving an end powder product with a particle size of less than 200 microns in 99% of cocoa powder, with a polyphenol content of more than 10% by weight of dry matter, and a fat content of less than 12% by weight as defined in Claim 1.

An optional phase of the process is that of the purification of the polyphenols obtained from the defatted cocoa cake or defatted meal (10-12% of fat) using an Amberlite column, whereby an end powder product with a particle size of less than 3 millimeters in 99%, preferably less than 0.5 millimeters, with a total polyphenol content comprised between 60% and 90% by weight of dry matter and a fat content of less than 12% by weight, is obtained.

Given the nature and objective of the new process, the selection of the starting product is important, because it will have a significant effect on the polyphenol content in the end product. In this sense, it must be emphasized that without actually forming part of the process, the best results will be obtained through a careful selection of the cocoa bean variety, for example, the CCN-51 clone of the Quevedo region (in Ecuador), selected due to its high polyphenol content.

The selection of the cocoa variety has been carried out according to the content of the polyphenols present in fresh cocoa fruit. A different cocoa variety refers not only to a different botanical variety but also to a subvariety from the crossing of different botanical varieties and further cultivated in a certain place. It is known that the same variety or subvariety cultivated in different origins produces cocoa seeds with different polyphenol content. Identical varieties within a same origin, according to the region in which they are produced, also provide cocoa seeds with different polyphenolic substance content.

The historical classification of cocoa comes from its relationship with Venezuela (Motamayor, 1997):
Criollo cocoa is native Venezuelan cocoa.

Trinitario cocoa is the cocoa from the crossing of Criollo cocoa with Amazon (Forastero) cocoa carried out in Trinidad Island. This species allowed repopulating the area with cocoas that were more resistant to diseases and more productive than Criollo cocoa after a natural disaster.

Forastero cocoa is cocoa from out of the country from the Amazon basin.

On the other hand, the International Trade Center (1991) defines cocoa types as:
"Criollo cocoa, the cocoa originally cultivated in the rain forests of Mexico, Central America and the north of South America. It produces medium to large beans (90 to 89 beans per 100 grams) with very light brown blackish ivory-colored cotyledons and a sweet cocoa aroma. They include porcelain, Merida, Guasare and original Chuao cocoas".

"Forastero cocoa comes from the upper parts of the Amazon basin, which produces small to medium beans (90 to 110 beans per 100 grams) of dark purple cotyledon". These varieties are responsible for the largest cocoa volume produced and reported in the world. The color difference existing between the cotyledons of Criollo and Forastero cocoa can be emphasized and is directly associated to the group of chemical substances called anthocyanins which are responsible for the development of the characteristic purple color of cocoa beans.

It has been shown that the amount of polyphenols in Criollo cocoa is approximately 2/3 of the amount present in Forastero cocoa (J. Wollgast, and E. Anklam, 2000).

Furthermore, as has already been indicated, according to the region in which they are produced, identical varieties provide very different types of beans giving rise to an enormous genetic variability of the cocoa species. This is due to the fact that within the same country and according to the cocoa producing region, as a result of the different types of intercrossing, different cocoa genotypes are developed giving rise to a wide genetic heterogeneity in the cocoa raw material. The case of beans from hybrid materials of Forastero cocoas with the influence of old Criollo and Trinitario cocoas; or the case of beans from Trinitario cocoas mixed with local Criollo cocoas from a certain region; or also the case of beans from a variety of Forastero, Trinitario and Criollo cocoas, among others, are examples. Each of these cocoa genotypes produced has certain characteristics including the bean size, the fermentation degree and the polyphenol content, for example. In an unpublished study carried out by the Natraceutical research group it has been shown that for unfermented and sun-dried seeds the total polyphenol content varies significantly according to the origin of the cocoa beans. Significant differences have also been found in the total polyphenol content within a single cocoa variety, from a single origin, according to the cocoa bean genotype.

Due to the foregoing, in order to carry out the process of the present invention, the CCN-51 clone from the Quevedo region in Ecuador has been selected as the most suitable starting product for the new process due to its high polyphenol content. It is a triple hybrid from the crossing of the Amazon IMC67 clone with the Trinitario ICS95 clone, which has in turn been crossed with the Amazon Canelo genotype, a regional genotype of the Quevedo area.

This triple hybrid contains a total polyphenol level of not less than 3% by weight in relation to the dry seed and not more than 14% by weight in relation to the dry cocoa seed. The total polyphenols were measured by the Folin-Ciocalteu method (Singleton and Rossi, 1965).

Another important aspect with respect to the raw material for elaborating a cocoa powder with high polyphenol content relates to the use of fresh unfermented cocoa seeds.

During the fermentation process, the intense modifications suffered by polyphenols start after the death of the seed, when the phenolic substances and enzymes are released from their respective cell storage areas. During this process, the soluble polyphenol and epicatechin content is reduced between approximately 10-20% (J. Wollgast, and E. Anklam). In another published study, the catechin losses after the fermentation process reached 90% (Kim and Keeney, 1984). In 1986, Pettipher estimated a 97% loss of phenolic compounds after four days of fermentation followed by the drying step.

Studies carried out by the Natraceutical research group show statistically significant differences in the total polyphenol content between the dry fermented seed and the dry unfermented seed, significantly higher total polyphenol levels being observed in the dry unfermented cocoa seeds than in the corresponding fermented seeds. It is thus confirmed that the fermentation process is a step exerting a significant effect in the reduction of phenolic compounds: catechin, epicatechin and procyanidins B1 and B2 of cocoa beans (unpublished data).

### Detailed Description of the Invention

The invention is defined by the appended claims.

The process for obtaining polyphenol-rich cocoa powder with low fat content specifically comprises eight main or essential phases, namely:
1.- Peeling the cocoa fruit;
2.- Depulping the seeds;
3.- Blanching the seeds, wherein the depulped cocoa is submersed in water until the internal part of the seed reaches a temperature between 85 and 100°C, which is maintained for a period between 3 and 15 minutes
4.- Drying the seeds;
5.- Defatting, wherein mechanical pressing is carried out by means of continuous expeller type presses at a temperature between 35°C and 85°C, until reducing the fat to less than 20% by weight;
6.- Stabilizing the defatted product, wherein the temperature of the defatted cocoa solid is reduced to less than 35°C in a continuous cooler with stirring blades;
7.- Grinding, and
8.- Micronizing
wherein the cocoa powder is obtained by means of a process in which, an intermediate cooling phase is carried out between the peeling phase and the depulping phase, in which the cocoa beans with their pulp are submersed in cold water at a temperature between 1°C and 20°C for a time period of no more than 3 hours; and
wherein the end powder product has a particle size of less than 200 microns in 99% of cocoa powder, with a phenol content of more than 10% by weight of dry matter and a fat content of less than 12% by weight;
and the following optional or secondary phases intercalated with the essential phases, namely:
4.1.- Cleaning the dry beans, and
4.2.- Shelling the dry beans, both between the drying and defatting phase
5.1.- Extracting and purifying the polyphenols of the cocoa extract obtained in the defatting phase
7.1.- Debacterizing the ground product, or
7.2.- Supercritically extracting the ground product with CO₂, both between the grinding and micronizing phase.

Each of the phases numbered according to the order of the process according to the invention is described below, in which the total of the possible phases is carried out:

### 1.- Peeling the cocoa fruit

This phase starts the process for obtaining polyphenol-rich cocoa powder with low fat content and during it, the cocoa fruit is open, the cocoa pulp is separated from its casing and the fresh unfermented beans are extracted from the fruit.

### Cooling the beans:

In this phase, the fresh unfermented cocoa beans are cooled by immersion in cold water. The temperature of the water must not be less than 1°C or more than 20°C.

The purpose of this phase is to prevent the complete or partial fermentation in the fresh cocoa beans by means of reducing the internal temperature of the fresh cocoa beans.

Additionally, the purpose of this process is to eliminate oxygen from the medium to delay to a large extent the start of the enzymatic browning reaction of fresh cocoa seeds and therefore, to reduce to a large extent the oxidation of the phenolic compounds of the fresh cocoa seeds.

The purpose of this process is also to eliminate 30% of the pulp surrounding the fresh cocoa seed for the sole purpose of optimizing the yield of the subsequent dry unfermented cocoa seed shelling process as well as its use for producing a cocoa solid with high polyphenol content.

In this phase, fresh unfermented beans are specifically submersed in water until a level in which it can be ensured that all the beans are covered by water, thus preventing the contact with oxygen in the air. The temperature of the water must not be more than 20°C and not less than 1°C. The maximum dwelling time of the beans submersed in water must not be more than 3 hours.

### 2.- Depulping the beans:

In this phase, the fresh cocoa beans are depulped for the purpose of separating between 35-40% of the pulp adhered to the fresh cocoa beans and optimizing the yield of the subsequent cocoa bean drying and shelling process. This depulping can be carried out with a stainless steel depulping machine with a sieve having a size between 3-5 mm.

### 3.- Blanching the beans:

In this phase, the cocoa beans are subjected to a blanching with water, at an internal temperature of the seed of not less than 85°C and not more than 100°C, for a time period of not less than 3 minutes and not more than 15 minutes.

This phase fulfills several functions, the first of which is to inactivate the endogenous activity of the PPO enzyme present in cocoa beans and obtain fresh unfermented cocoa seeds, either without PPO activity or with a reduced PPO activity. The second function is to completely or partially preserve the initial content of the phenolic compounds initially present in fresh cocoa beans, these phenolic compounds being quantified by their total polyphenol content and their use as raw material for producing cocoa solids with high total polyphenol content. The third function is to prevent to a large extent the reduction of the monomers, catechin and epicatechin, and the dimers, procyanidin B1 and procyanidin B2 of cocoa beans in subsequent cocoa processing steps, to finally obtain a cocoa powder with high concentrations of the monomers, catechin, epicatechin, and of the dimers procyanidin B1 and B2 (quantified by HPLC with a Diode Array detector). And the fourth function is to reduce the post-harvest enzymatic deterioration in fresh cocoa beans, leading to the loss of the initial color of the cocoa seeds, and to reduce the initial microbial load of fresh cocoa beans, increasing the useful life of dry unfermented cocoa beans during their storage. Therefore, violet, dry unfermented cocoa beans are produced during this phase for their use as raw material in the production of violet cocoa solids with high polyphenol content.

For the purpose of inactivating the PPO enzyme, the most favorable conditions for this inactivation have been studied in fresh cocoa beans by means of a heat treatment with water (blanching). Nine tests at three different temperatures and times were carried out for the experimental design of this study, taking the degree of melanosis developed after adding the catechol reagent in the fresh cocoa seeds as the response surface. A 0 to 10 color scale was established to describe the progression of melanosis (Pons et al.).

In a particular study carried out, it has been verified that after the fresh cocoa bean blanching process at a temperature of 95°C and for a time period of 5 minutes, during the drying process the degradation of the monomer epicatechin is reduced by 21%, the degradation of the monomer catechin is reduced by 38% and the degradation of the dimer procyanidin B2 is reduced by 20% (measurements carried out on dry unfermented cocoa beans).

### 4.- Drying the beans:

In this phase, the unfermented cocoa seeds with a reduced polyphenol oxidase enzyme activity are dried at a temperature of less than 80°C to obtain dry unfermented cocoa seeds with high polyphenol content and low moisture content <=9% by dry seed weight.

The drying process can be carried out at room temperature or at high temperatures and preferably for a time period such that a significant amount of the content of the polyphenols initially present in the unfermented cocoa seed is preserved.

The purpose of the drying process is to reduce the moisture content of the cocoa seed to below 9% by dry weight, preferably below 7% by dry weight, and more advantageously below 3%.

### 4.1.- Cleaning the beans (optional):

In this phase, the dry unfermented cocoa seeds are cleaned to eliminate the foreign substances associated thereto.

This phase is carried out mechanically with the use of a sieve between 2 and 10 mm.

### 4.2.- Shelling the beans (optional):

This phase is in any case prior to the pressing phase. In this phase, the shell associated to the bean is separated and the cocoa nibs are obtained, the term nibs referring to shell-free cocoa solids.

The cocoa shell contained by cocoa seeds is eliminated therefrom to produce cocoa nib fragments. The conventional bean shelling process includes the bean breaking process giving rise to a mixture of pieces of nibs and shell which causes the separation of the shell from the nib by means of a sieving process with a suction system.

Conventional equipment for shelling cocoa beans, such as those manufactured by Martin Lloveras, Bauermeister, Lehmann and others, achieve yields of 1 % by weight of residual shell on the nib.

In the shelling, the nibs must preferably contain less than 5% by weight of residual shell, preferably less than 2% by weight, and more preferably less than 1% by weight.

### 5.- Defatting:

In this phase, the cocoa seeds are defatted by means of a continuous mechanical pressing using expellers.

The "ejection" press (known as expeller), also called "extruder" or "screw" press, is a continuous mechanical extractor, in which the cocoa fat is squeezed from the raw material in a single step under high pressure. The fundamental difference with hydraulic presses lies in the fact that the latter are usually not continuous.

This pressing equipment consists of a continuous helical screw rotating from a perforated static cylinder. When the material is transported along the length of the cylinder, the pressure increases causing the fat to be "expelled" and drained through small grooves. The compressed and defatted material, called cake (if it comes from nibs) or meal (if it comes from unshelled seeds) is unloaded at one end and the fat is collected at the base of the press.

The purpose of this phase is to defat the cocoa seeds from approximately 50% by weight of fat to less than 12%, and more advantageously less than 8%; carrying out the defatting process at a temperature of not less than 35°C and during which the temperature of the defatted cake or meal after the pressing will not be more than 85°C. An unfermented cocoa meal or cake, defatted to below 12% by weight of fat content from unfermented cocoa seeds and with a reduced polyphenol oxidase activity, is thus achieved.

The term cake refers to the cocoa solid with less than 2% of residual shell and with a residual fat content of less than 20% by weight.

The term meal refers to the cocoa solid containing 100% of the shell associated to cocoa beans, and with a residual fat content of less than 20% by weight.

### 5.1.- Extracting and purifying polyphenols from the cocoa material obtained in the defatting phase

Polyphenols are a mixture of substances with very different molecular weights and polarity. It is known that they are soluble or partially soluble in polar solvents or their mixtures (for example water, methanol, ethanol, acetone, ethyl acetate). Methods using ion exchange resins and absorption resins, such as for example counterflow or normal silica gel resins, or by gel filtration, have been used for their purification, these methods being able to be used alone or in combination.

The polyphenol purification method disclosed herein uses amberlite resin (macroreticular aromatic polymer) as a selective adsorbent and which can be tolerated in the food industry, specifically Amberlite™FPX66 (Rohm and Haas).

There is a review on the extraction and analysis of phenolic derivatives (Marian Naczk, Fereidoon Shahidi; Journal of Chromatography A, 1054 (2004) 95-111) in which section 3.1 (Liquid-solid phase procedures) mentions that Amberlite XAD-2 particles have been used for isolating and purifying phenolic products from aqueous plant extracts and acidified aqueous honey solutions. The Amberlite particles were stirred for 4 hours with the aqueous extracts and the column was then packed with the mixture.

Document WO 01/78859 A1 describes a method for obtaining useful materials from the byproducts of fruit and vegetable processing. Example 1 describes the separation of polyphenols of pectin (in this case useful material) using an Amberlite XAD 16HP column.

Disclosed herein is a cocoa extract obtained by means of a hydroalcoholic extraction, which extract is later purified by means of a column purification process, for which an adsorption resin (Amberlite™FPX66 resin) is used.

### 5.1.1 Obtaining the extract

The extract is obtained from a solid-liquid extraction process from the cocoa material obtained from the expeller and using 40-95%, preferably 55-80%, more preferably 60% isopropanol as a solvent for carrying out the extraction. The solid-liquid ratio is 1/4 and the extraction process is repeated as many times as necessary until using up the residue as much as possible. Said extraction process has occasionally been repeated 4 to 6 times. The obtained extract is filtered under vacuum and was distilled at 70°C for example until the elimination of isopropanol; it is then subjected to the column purification process using amberlite resin as the packing.

### 5.1.2 Purification of the extract in a column using an amberlite packing

The aqueous extract obtained after the elimination of isopropanol is filtered under vacuum and is subjected to a purification process using an adsorption resin. One type of resin used which is suitable for polyphenol adsorption is amberlite resin (AMBERLITE FP*X66) of the Rohm and Haas company. Other amberlite resins with similar features can logically be used.

The steps to be followed for purifying polyphenols in a column are:
1. Packing the column (for example, 60 g of resin per column). It consists of introducing the adsorption resin inside the column preventing the formation of air pockets between its particles to thus obtain a uniform bed.
2. Washing the column with distilled water.
3. Loading step: the solution to be treated is introduced in the column and flows through the adsorption resin, the polyphenols to be purified being adsorbed in the resin.
4. Washing with water.
5. Eluting the polyphenols with 70-80% aqueous isopropanol.

The compounds of interest retained in the column are eluted.
6. Washing the column.

The purified extract fraction is distilled and the aqueous extract is dried under vacuum, a dark red powder being obtained.

A cocoa product with a polyphenol profile comprising 550-1100 mg/g of total polyphenols is obtained by means of this method (modified Folin-Ciocalteu method, 1965). The specific values of catechin: 25-60 mg/g, epicatechin: 140-300 mg/g, procyanidin B1: 1.5-15 mg/g, and procyanidin B2: 60-120 mg/g (the quantification of the monomers catechin and epicatechin and dimers of procyanidin B1 and B2, has been carried out by means of high performance liquid chromatography with a diode array detector (HPLC-DAD). ;*FP:Food Processing

### 6.- Stabilizing the defatted product:

In this phase, the defatted unfermented cocoa solids are cooled to a temperature less than 35°C at the exit of the presses, to stabilize them and optimize the yield of the subsequent grinding process. This stabilization process is carried out in a continuous cooler with stirring blades, such as in stainless steel blade cooler. This cooler, which is internally equipped with blades stirring the product, is provided with a sleeve through which cold water is circulated and inside of which cold air is circulated.

### 7.- Grinding:

In this phase, the unfermented cocoa solids which are defatted to below 20% by weight are ground to a particle size of less than 0.5 mm, i.e. 500 microns.

This grinding can be carried out in a hammer mill provided with a classifier which allows adjusting the particle size.

The purpose of this phase is to obtain an optimal solid granulometry so that the subsequent sterilization treatment of the product is homogeneous and effective. After the grinding process, 80% of the particles must preferably have a size of less than 500 microns, preferably 99% of the ground products must contain a particle size of less than 500 microns, and more preferably 99% of the ground products must contain a particle size of less than 500 microns.

### 7.1.- Debacterization or heat treatment (optional):

In this phase, the microbial load of the ground products with a particle size of less than 500 microns is reduced by means of a disinfection process with humid heat and during which the reduction of the total polyphenol content of the heat-treated cocoa products with respect to the ground products with a particle size of less than 500 microns is less than 30%, advantageously less than 1.5%; these total polyphenols being quantified by the Folin-Ciocalteu method.

The purpose of this process is to achieve that the heat-treated cocoa products comply with the parameters considered in the legal specifications defined for a cocoa powder, for the purpose of achieving a microbiologically acceptable level for the consumer in the end product. For example, the specifications defined for a cocoa powder: Total count of aerobic mesophiles <=5000 cfu/g, Molds<=100 cfu/g, Yeasts<= 100 cfu/g, Enterobacteriaceae <=10 cfu/g, absence of E Coli /g and absence of Salmonella /25 g.

The purpose of this process is also to reduce the possible microbiological deterioration mechanisms of the final solid developed, to increase the useful life time during its storage. Specifically, during this phase, the ground product with a percentage of fat by weight of less than 12% and with a particle size of less than 500 microns is loaded in horizontal trays and then placed in an autoclave where the debacterization of the product by a steam jet takes place. The fixed debacterization conditions whereby the microbiological parameters of the total count of aerobic mesophiles <=5000 cfu/g, Molds<=100 cfu/g, Yeasts <= 100 cfu/g, Enterobacteriaceae <=10 cfu/g, absence of E Coli /g and absence of Salmonella 125 g have been reached have been 121°C for 1 minute. In these conditions, the obtained product is agglutinated, having a physical "block" appearance, therefore it is introduced in a mill for example a mill with blades until obtaining a heat-treated unfermented cocoa solid with a particle size of less than 4 mm and with microbiological parameters suitable for human consumption, for example: Total Count of aerobic mesophiles <=5000 cfu/g, Molds<=100 cfu/g, Yeasts<= 100 cfu/g, Enterobacteriaceae <=10 cfu/g, absence of E Coli /g and absence of Salmonella/25 g.

### 7.2.- Supercritical extraction (optional):

In this phase, the cocoa raw materials are defatted using CO₂ in supercritical conditions as an extraction solvent.

The cocoa materials to be defatted according to the process described in the present invention are the ground cocoa cake below 500 microns, the ground cocoa meal of 500 microns, as well as their heat-treated cocoa products and with a particle size of less than 4 mm (4000 microns).

The products obtained as a result of the defatting process with supercritical CO₂ are denominated low-fat products.

After the extraction using CO₂ in supercritical conditions, the residual fat content of the aforementioned products must be less than 5% by weight of fat, preferably less than 3% by weight of fat, and more advantageously less than 1% by weight of fat.

It must be indicated that low-fat products resulting from the extraction using CO₂ in supercritical conditions and which have not been heat-treated in the prior debacterization process can be heat-treated as described previously in the debacterization phase to reduce the microbial load.

### 8.- Micronizing:

The purpose of this phase is to obtain 99% of cocoa powder with a particle size of less than 200 microns, preferably less than 100 microns and more advantageously less than 75 microns.

This micronization can be carried out in a classifier mill.

According to the process described in the present invention, the cocoa materials that must be ground are ground products with a particle size of less than 500 microns (0.5 mm), corresponding heat-treated cocoa products with a particle size of less than 4000 microns (4 mm), low-fat products as well as heat-treated low-fat cocoa products.

The end products obtained as a result of this grinding process and containing a particle size of less than 75 microns are denominated cocoa powders with high polyphenol content.

### Cocoa products

According to that described, the reduction of the phenolic compounds initially present in fresh cocoa beans is prevented by using the different alternatives mentioned in the process object of the invention, and on the other hand, the fat content is reduced until achieving an end powder product with high polyphenol content, a reduced fat content and 99% of particles with the desired size.

Specifically, as described herein, cocoa powders can be obtained with the steps of the process described in the claims, which cocoa powders have a polyphenol content of more than 10% in a dry base, preferably more than 12% in a dry base, more preferably more than 14% in a dry base, more preferably more than 16% in a dry base, and more preferably more than 25% in a dry base; and a fat content of less than 12% by weight, more preferably less than 8%, more preferably less than 5%, and more preferably less than 1%.

And as also described herein, cocoa powder extracts can be obtained with the treatment described herein which cocoa powder extracts have a content of 60% to 90% of polyphenols, preferably of 75% to 85% by weight of dry matter; alternatively with a fat content of less than 12%, preferably less than 3%.

The particle size can be according to the cases and the intended use of the cocoa powder obtained, of less than 4 mm, preferably less than 500 microns, less than 200 microns, less than 100 microns or less than 75 microns.

Likewise, the water content in all these products can be less than 9%, preferably less than 7% and more preferably less than 5% depending on the intended use of the product.

### EXAMPLES

### Example 1. Process for obtaining cocoa powder with a content between 10 to 12% of fat by weight.

### A) Obtaining cocoa cake:

The CCN51 clone of the Amazon variety from the Quevedo region in Ecuador was used as cocoa as raw material.

10,000 Kg of fresh beans are cooled in 30,000 Kg of water at 15°C for the purpose of obtaining cocoa powder with high total polyphenol content as well as its monomers (catechin and epicatechin) and of its dimers (procyanidins B1 and B2). This operation is carried out immediately after opening the pods for the purpose of preventing fermentation.

After 2 hours, the pulp is removed using a TALSA stainless steel sieving machine (2 mm sieve), and approximately 7,000 kg of depulped beans are obtained.

Immediately afterwards, the pulp-free beans are subjected to a blanching treatment in boiling water until achieving an internal bean temperature of 95°C, from which, the dwelling time of the beans in water is of 5 minutes, after which the beans are passed through a sieve for the purpose of removing the surface water therefrom.

The beans are then dried in a continuous web dryer with an air stream at 70°C until the bean moisture content is approximately 5%, around 3,150 kg of dry beans being obtained.

These beans are then subjected to cleaning process in a Buhler to eliminate the remains of foreign particles, and later to a shelling process (DK 1000 Martin Lloveras), after which approximately 2,700 kg of cocoa nibs being obtained. This process takes 10 hours.

The nibs are then heated up to 55°C and then they are subjected to a partial separation from butter by means of a mechanical pressing using a continuous expeller (KP Harburguer Einsem Bronzewerke), the temperature of the cake at the exit of the press being 65°C. Approximately, 1,500 kg of "cocoa cake" are thus obtained with a fat content of 11 % by weight.

### B) Obtaining cocoa powder from cocoa cake:

The cake obtained in the previous section (1A) is stabilized in a rotary blade cooler (Ingeletsa) for the purpose of subsequently being ground. The temperature of the cake at the exit of the cooler is 15°C.

The cake grinding process is carried out in a hammer mill provided with a sieve (MS-33-M1 Gruber Hnos. S.A.). After the grinding, 99% of the particles have a size of less than 500 microns.

The ground cake is subjected to a debacterization process in an industrial autoclave (Steamlab) by means of a heat treatment at 121°C for 3 minutes. After the heat treatment, the product agglutinates showing a solid aspect in the form of a block, which is ground in a blade mill until obtaining a solid with a particle size of less than 4 mm (4000 microns).

Finally, the cocoa solid is micronized in a classifier mill (MS 1000 Micron Process) until obtaining a powder in which 99% of particles is less than 75 microns.

Approximately 1,425 kg of cocoa powder with 11% of fat and 99% of particular less than 75 microns are thus obtained.

### Example 2. Process for obtaining cocoa powder with a content of less than 1% (0.9%) of dry matter by weight.

This process includes all the previous processes, with the exception that before the final micronization, the cocoa solids are defatted using CO₂ in supercritical conditions as a solvent for the extraction of cocoa butter. To that end, the sterilized solid with a particle size of less than 4 mm is put in contact with CO₂ in supercritical conditions, this extraction being carried out at temperatures of no more than 60°C. After separating the butter, 1,328 Kg of defatted cocoa solid are obtained.

Finally, like in example 1, the defatted cocoa solids are micronized in a classifier mill (MS 1000 Micron Process) until 99% of the particles have a size of less than 75 microns. Approximately 1,262 Kg of cocoa powder with 99% of particles with a size of less than 75 microns and with a fat content of 0,9% by weight are thus obtained.

### Example 3. Microbiological profile of the cocoa solids after sterilization.

Example 1 describes the debacterization process, among others. Table 1 shows the microbiological profile before and after the heat treatment applied to the ground cocoa cake with a particle size of less than 0.5 mm (500 microns).

### TABLE 1

**TABLE 1**

| Method | control (ground cake before debacterization) | DEBACTERIZED solid At 121°C/3 min (particle size of less than 4 MM) |
|---|---|---|
| Total aerobe count | | |
| Mesophiles (cfu/g)/ Ph. Eu. | 180000000 | <10 |
| ASU L 00.00-88 | | |
| Yeasts (cfu/g)/Ph.Eu | | |
| YGC-Agar/25°C/120h | <10 | <10 |
| LOD=10/g | | |
| Molds (cfu/g)/ASU L 00.00-88 | 1300 | <10 |
| Enterobacteriaceae (NMP/g) | | |
| ASU L 00.00-88 | 220000 | <10 |
| E Coli (cfu/g)/ | | |
| TBX-Agar/44°C/20h LOD=10/G | <10 | <10 |
| Salmonella/25g/ASU L00.00-20 | Neg. | Neg. |
| Heat-resistant sporeformers | | |
| Aerobic mesophiles (cfu/g) | | |
| PCA/30°C/72h LOD=10/g | 710000 | <10 |
| SGS MIB M 1.15 | | |
| Heat-resistant sporeformers | | |
| Aerobic thermophiles (cfu/g) | | |
| PCA/55°C/72h LOD=10/g | 210000 | <10 |
| SGS MIB M 1.28 | | |

### Example 4.

Table 2 shows the polyphenol content of a natural non-alkalized cocoa powder which is available on the market and of the products obtained in examples 1 and 2.

The quantification of the polyphenols has been carried out by the modified Folin-Ciocalteu method (Singleton, V.; Rossi, J. Colorimetry of total phenolics with phosphomolybdicphosphotungstic acid reagents. Am. J. Enol. Vitic. 16, 144-158, (1965) and the quantification of monomers (catechin and epicatechin) and dimers of procyanidin B1 and B2, has been carried out by means of high performance liquid chromatography with a diode array detector (HPLC-DAD). The results are in mg/g in a dry base.

**TABLE 2**

| Polyphenols mg/g (d.b.)¹ | Standard Natural Cocoa powder | Example 1: 11% dry matter by weight | Example 2: 0,9% dry matter by weight |
|---|---|---|---|
| ⁽²⁾ Catechin | 0.32 | 5.03 | 5.26 |
| ⁽²⁾ Epicatechin | 1.20 | 20.19 | 25.60 |
| ⁽²⁾ Procyanidin B1 | 0.10 | 1.17 | 1.07 |
| ⁽²⁾ Procyanidin B2 | 0.00 | 12.64 | 12.40 |
| ⁽³⁾ Total | 53 | 183.4 | 197 |

| | | | |
|---|---|---|---|
| <(1)>mg of polyphenols/ g of product measured in a dry base. <(2)>Analysis carried out by HPLC with a Diode Array detector <(3)>Analysis carried out by the Folin-Ciocalteu method | | | |

### Reference Example 5. Polyphenol extraction from cocoa cake using an adsorption resin

The raw material used was a cocoa cake with 15.27 % by weight of fat.

400 g of cocoa cake were mixed with 1600 mL of 60% aqueous isopropanol with magnetic stirring for 1 hour. The separation of the solid was carried out by means of vacuum filtration using a filter with a diameter of 20 cm. The obtained solid was again extracted 2 more times following the same extraction process.

The combination of the obtained extracts was subjected to a distillation in a rotary evaporator at 70°C and 120 mbar until the complete elimination of isopropanol. A final volume of 860 mL of an aqueous extract which was subsequently purified was obtained.

Two columns in series with an internal diameter of 24 mm are each packed with 60 g of amberlite resin (Amberlite™FPX66 of Rohm and Haas) until reaching a bed height of 21 cm (Bed volume BV=95 ml per column). The column is packed following the manufacturer's instructions consisting of introducing the adsorption resin inside the column preventing the formation or air pockets between its particles to thus obtain a uniform bed. The resin is washed with 500 mL of demineralized water with a flow rate of 800 ml/h (4.3 BV/ h).

Two 430 ml portions of the aqueous extract obtained are passed through each column filled with 95 mL of adsorbent resin (Amberlite™FPX66 and at a flow of 3.2 bed volumes /h. In this step, the aqueous extract flows through the resin, the polyphenols being adsorbed and retained in the resin of the column.

The two columns in series are washed with 1000 mL of demineralized water and with a flow of 600 ml/h (3.2 BV/h) to eliminate the more polar compounds which are not polyphenols and are retained on the column.

The polyphenols retained on the column will then be desorbed. The polyphenols are eluted with 600 ml of 74% aqueous isopropanol with a flow rate of 600 ml/h (3.2 BV/h). The purified extract fraction obtained is denominated eluate (a).

The column is washed with 900 ml of demineralized water with a flow rate of 600 ml/h (3.2 BV/h). The first aqueous 500 ml obtained are called eluate (b).

The purified extract fractions, eluate (a) and eluate (b) from the purification of both aqueous extract portions, are pooled, a final volume of 2200 mL being obtained.

The purified extract is distilled and concentrated in a rotary evaporator at 70°C and 120 mbar, 73 grams of a viscous 45° Brix concentrate being obtained.

The isopropanol-free concentrate is finally vacuum dried at 70°C and 15 mbar for 2 hours, 32 grams of a dry purified extract being obtained (8% mass yield), which is subsequently ground until a dark red granulated powder is obtained.

The total polyphenol content contained in the granulated powder is of 843 mg/g (results expressed in a dry base).

Table 3 shows the polyphenol content of the cocoa cake which has been used as a starting product for the extraction of polyphenols and their purification using an adsorption resin, and of the product obtained in Example 5.

The quantification of the polyphenols has been carried out by the modified Folin-Ciocalteu method (Singleton, V.; Rossi,J. Colorimetry of total phenolics with phosphomolybdic-phosphotungstic acid reagents. Am. J. Enol. Vitic. 16, 144-158, (1965) and the quantification of the monomers (catechin and epicatechin) and dimers of procyanidin B1 and B2, has been carried out by means of high performance liquid chromatography with a diode array detector (HPLC-DAD). The results are in mg/g in a dry base.

**TABLE 3**

| Polyphenols mg/g (d.b.)¹ | Cocoa purified in Amberlite column | Cocoa cake: 15.27% of fat by weight | Example 6: Amberlite column |
|---|---|---|---|
| ⁽²⁾ Catechin | 25-60 | 3.5 | 41.55 |
| ⁽²⁾ Epicatechin | 140-300 | 15.64 | 233 |
| ⁽²⁾ Procyanidin B1 | 1.5-15 | 2.10 | 5.51 |
| ⁽²⁾ Procyanidin B2 | 60-120 | 9.61 | 85.32 |
| ⁽³⁾ Total | 550-1100 | 1 76 | 843 |

| | | | |
|---|---|---|---|
| <(1)>mg of polyphenols/ g of product measured in a dry base. <(2)>Analysis carried out by HPLC with a Diode Array detector <(3)>Analysis carried out by the Folin-Ciocalteu method | | | |

## Claims

1. A process for producing a polyphenol-rich cocoa powder with low fat content comprising:
1.- Peeling the cocoa fruit;
2.- Depulping the seeds;
3.- Blanching the seeds, wherein the depulped cocoa is submersed in water until the internal part of the seed reaches a temperature between 85 and 100°C, which is maintained for a period between 3 and 15 minutes;
4.- Drying the seeds;
5.- Defatting, wherein mechanical pressing is carried out by means of continuous expeller type presses at a temperature between 35°C and 85°C, until reducing the fat to less than 20% by weight;
6.- Stabilizing the defatted product, wherein the temperature of the defatted cocoa solid is reduced to less than 35°C in a continuous cooler with stirring blades;
7.- Grinding;
8.- Micronizing;
wherein the cocoa powder is obtained by means of a process in which, an intermediate cooling phase is carried out between the peeling phase and the depulping phase, in which the cocoa beans with their pulp are submersed in cold water at a temperature between 1°C and 20°C for a time period of no more than 3 hours; and
wherein the end powder product has a particle size of less than 200 microns in 99% of cocoa powder, with a phenol content of more than 10% by weight of dry matter and a fat content of less than 12% by weight.

2. A process for producing cocoa powder according to claim 1, wherein in step 2 the cocoa is depulped by means of a sieve with a size of 3 to 5mm.

3. A process for producing a cocoa powder according to any of claims 1 or 2, wherein in step 4, the depulped cocoa is heated at a temperature between room temperature and 80°C, preferably less than 50°C, until its water content reaches between 3 % and 9 % dry seed weight, and preferably 7 % by weight in a dry seed base.

4. A process for producing a cocoa powder according to any of claims 1 to 3, wherein in step 7, the stabilized cocoa is subjected to the action of a mill to obtain at least 99% of cocoa powder with a particle size of less than 500 microns, preferably of less than 200 microns, and most preferably of less than 75 microns.

5. A process for producing a cocoa powder according to any of claims 1 to 4, wherein a bean cleaning phase is carried out between the drying phase and the defatting phase, in which the depulped and dry cocoa beans are subjected to mechanical cleaning by sieving, with a sieve between 2 and 10 mm.

6. A process for producing a cocoa powder according to any of claims 1 to 5, wherein a bean shelling phase is carried out between the drying phase and the defatting phase, in which the dry cocoa beans are shelled by means of conventional equipment until an amount of residual shell of less than 5% by weight of the shelled beans, preferably of less than 2% by weight of shelled beans and more preferably less than 1% by weight of shelled beans.

7. A process for producing a cocoa powder according to any of claims 1 to 6, wherein an intermediate debacterization phase is carried out between the grinding phase and the micronizing phase, in which the ground cocoa with a particle size of less than 500 microns is introduced in an autoclave at a temperature between 90°C and 125°C, between 30 seconds and 4 minutes, preferably at a temperature of around 121°C for 1 minute, an agglutinated product remaining after the treatment which is introduced in a mill until reaching a particle size of less than 4mm.

8. A process for producing a cocoa powder according to any of claims 1 to 7, wherein a supercritical extraction phase is carried out between the grinding phase and the micronizing phase, in which the ground cocoa is subjected to a treatment with CO₂ in supercritical conditions, until achieving a fat weight of less than 5%, preferably of less than 3% and more preferably less than 1%.

## Patentansprüche

1. Vorgang zum Erzeugen eines polyphenolreichen Kakaopulvers mit geringem Fettgehalt, Folgendes umfassend:
1.- Schälen der Kakaofrucht;
2.- Entfleischen der Samen;
3.- Blanchieren der Samen, wobei der entfleischte Kakao in Wasser getaucht wird, bis der innere Teil des Samens eine Temperatur zwischen 85 und 100 °C erreicht, die für einen Zeitraum zwischen 3 und 15 Minuten aufrechtgehalten wird;
4.- Trocknen der Samen;
5.- Entfetten, wobei ein mechanisches Pressen mittels Pressen der kontinuierlichen Austreiberart bei einer Temperatur zwischen 35 °C und 85 °C durchgeführt wird, bis das Fett auf weniger als 20 Gew.-% gesenkt ist;
6.- Stabilisieren des entfetteten Erzeugnisses, wobei die Temperatur des entfetteten Kakaofeststoffs in einem Durchlaufkühler mit Rührblättern auf weniger als 35 °C gesenkt wird;
7.- Mahlen;
8.- Mikronisieren;
wobei das Kakaopulver mittels eines Vorgangs erhalten wird, in dem eine Zwischenkühlphase zwischen der Schälphase und der Entfleischungsphase durchgeführt wird, in der die Kakaobohnen mit ihrem Fruchtfleisch in kaltes Wasser bei einer Temperatur zwischen 1 °C und 20 °C für einen Zeitraum von höchstens 3 Stunden getaucht werden; und
wobei das Endpulvererzeugnis eine Teilchengröße von weniger als 200 Mikrometern in 99 % Kakaopulver mit einem Phenolgehalt von mehr als 10 Gew.-% Trockensubstanz und einem Fettgehalt von weniger als 12 Gew.-% aufweist.

2. Vorgang zum Erzeugen des Kakaopulvers nach Anspruch 1, wobei der Kakao in Schritt 2 mittels eines Siebs mit einer Größe von 3 bis 5 mm entfleischt wird.

3. Vorgang zum Erzeugen eines Kakaopulvers nach einem der Ansprüche 1 oder 2, wobei der entfleischte Kakao in Schritt 4 bei einer Temperatur zwischen Raumtemperatur und 80 °C, bevorzugt weniger als 50 °C, erwärmt wird, bis sein Wassergehalt zwischen 3 % und 9 % Trockensamengewicht und bevorzugt 7 Gew.-% in einer Trockensamenbasis erreicht.

4. Vorgang zum Erzeugen eines Kakaopulvers nach einem der Ansprüche 1 bis 3, wobei der stabilisierte Kakao in Schritt 7 der Einwirkung einer Mühle unterzogen wird, um wenigstens 99 % Kakaopulver mit einer Teilchengröße von weniger als 500 Mikrometern, bevorzugt von weniger als 200 Mikrometern und am stärksten bevorzugt von weniger als 75 Mikrometern, zu erhalten.

5. Vorgang zum Erzeugen eines Kakaopulvers nach einem der Ansprüche 1 bis 4, wobei eine Bohnenreinigungsphase zwischen der Trocknungsphase und der Entfettungsphase durchgeführt wird, in der die entfleischten und trockenen Kakaobohnen mechanischer Reinigung durch Sieben mit einem Sieb zwischen 2 und 10 mm unterzogen werden.

6. Vorgang zum Erzeugen eines Kakaopulvers nach einem der Ansprüche 1 bis 5, wobei eine Bohnenenthülsungsphase zwischen der Trocknungsphase und der Entfettungsphase durchgeführt wird, in der die trockenen Kakaobohnen mittels herkömmlicher Einrichtung bis zu einer Restschalenmenge von weniger als 5 Gew.-% der enthülsten Bohnen, bevorzugt von weniger als 2 Gew.-% der enthülsten Bohnen und stärker bevorzugt von weniger als 1 Gew.-% der enthülsten Bohnen, enthülst werden.

7. Vorgang zum Erzeugen eines Kakaopulvers nach einem der Ansprüche 1 bis 6, wobei eine Zwischenentkeimungsphase zwischen der Mahlphase und der Mikronisierungsphase durchgeführt wird, in der der gemahlene Kakao mit einer Teilchengröße von weniger als 500 Mikrometern in einem Autoklaven bei einer Temperatur zwischen 90 °C und 125 °C, zwischen 30 Sekunden und 4 Minuten, bevorzugt bei einer Temperatur von etwa 121 °C 1 Minute lang, eingeführt wird, wobei ein agglutiniertes Erzeugnis nach der Behandlung verbleibt, das in eine Mühle eingeführt wird, bis eine Teilchengröße von weniger als 4 mm erreicht ist.

8. Vorgang zum Erzeugen eines Kakaopulvers nach einem der Ansprüche 1 bis 7, wobei eine Phase überkritischer Extraktion zwischen der Mahlphase und der Mikronisierungsphase durchgeführt wird, in der der gemahlene Kakao einer Behandlung mit CO₂ unter überkritischen Bedingungen unterzogen wird, bis ein Fettgewicht von weniger als 5 %, bevorzugt von weniger als 3 % und stärker bevorzugt von weniger als 1 % erzielt wird.

## Revendications

1. Procédé de production d'une poudre de cacao riche en polyphénols avec une faible teneur en matières grasses comprenant :
1.- L'épluchage de la cabosse ;
2.- Le dépulpage des fèves ;
3.- Le blanchissement des fèves, le cacao dépulpé étant immergé dans de l'eau jusqu'à ce que la partie interne de la fève atteigne une température comprise entre 85 et 100 °C, qui est maintenue pendant une durée comprise entre 3 et 15 minutes ;
4.- Le séchage des fèves ;
5.- Le dégraissage, le pressage mécanique étant effectué au moyen de presses de type presse à vis à une température comprise entre 35 °C et 85 °C, jusqu'à ce que la teneur en matières grasses soit réduite à moins de 20 % en poids ;
6.- La stabilisation du produit dégraissé, la température du solide de cacao dégraissé étant réduite à moins de 35 °C dans un refroidisseur continu à lames d'agitation ;
7.- La mouture ;
8.- La micronisation ;
la poudre de cacao étant obtenue au moyen d'un procédé dans lequel une phase de refroidissement intermédiaire est effectuée entre la phase d'épluchage et la phase de dépulpage, dans laquelle les graines de cacao avec leur pulpe sont immergées dans de l'eau froide à une température comprise entre 1 °C et 20 °C pour une durée ne dépassant pas 3 heures ; et
le produit en poudre final ayant une granulométrie inférieure à 200 microns dans 99 % de cacao en poudre, avec une teneur en phénol supérieure à 10 % en poids de matière sèche et une teneur en matières grasses inférieure à 12 % en poids.

2. Procédé de production d'une poudre de cacao selon la revendication 1, dans lequel à l'étape 2 le cacao est dépulpé au moyen d'un tamis avec une taille de 3 à 5 mm.

3. Procédé de production d'une poudre de cacao selon l'une quelconque des revendications 1 ou 2, dans lequel, à l'étape 4, le cacao dépulpé est chauffé à une température comprise entre la température ambiante et 80 °C, de préférence inférieure à 50 °C, jusqu'à ce que sa teneur en eau atteigne entre 3 % et 9 % en poids de fèves sèches, et de préférence 7 % en poids dans une base de fèves sèches.

4. Procédé de production d'une poudre de cacao selon l'une quelconque des revendications 1 à 3, dans lequel, à l'étape 7, le cacao stabilisé est soumis à l'action d'un broyeur pour obtenir au moins 99 % de cacao en poudre ayant une granulométrie inférieure à 500 microns, de préférence inférieure à 200 microns et plus préférablement inférieure à 75 microns.

5. Procédé de production d'une poudre de cacao selon l'une quelconque des revendications 1 à 4, dans lequel une phase de nettoyage des graines est effectuée entre la phase de séchage et la phase de dégraissage, dans laquelle les graines de cacao sèches et dépulpées sont soumises à un nettoyage mécanique par tamisage, avec un tamis compris entre 2 et 10 mm.

6. Procédé de production d'une poudre de cacao selon l'une quelconque des revendications 1 à 5, dans lequel une phase de décorticage des graines est effectuée entre la phase de séchage et la phase de dégraissage, dans laquelle les graines de cacao sèches sont décortiquées au moyen d'un équipement conventionnel jusqu'à une quantité de coque résiduelle inférieure à 5 % en poids des graines décortiquées, de préférence inférieure à 2 % en poids de graines décortiquées et plus préférablement inférieure à 1 % en poids de graines décortiquées.

7. Procédé de production d'une poudre de cacao selon l'une quelconque des revendications 1 à 6, dans lequel une phase de débactérisation intermédiaire est effectuée entre la phase de mouture et la phase de micronisation, dans laquelle le cacao moulu avec une granulométrie inférieure à 500 microns est introduit dans un autoclave à une température comprise entre 90 °C et 125 °C, entre 30 secondes et 4 minutes, de préférence à une température d'environ 121 °C pendant 1 minute, un produit agglutiné restant après le traitement qui est introduit dans un broyeur jusqu'à atteindre une granulométrie inférieure à 4 mm.

8. Procédé de production d'une poudre de cacao selon l'une quelconque des revendications 1 à 7, dans lequel une phase d'extraction supercritique est effectuée entre la phase de mouture et la phase de micronisation, dans laquelle le cacao moulu est soumis à un traitement avec du CO₂ dans des conditions supercritiques, jusqu'à atteindre un poids de graisse inférieur à 5 %, de préférence inférieur à 3 % et plus préférablement inférieur à 1 %.
